# EUROPEAN PATENT APPLICATION

(11) **EP 4 458 365 A1**
(43) Date of publication of application: **06.11.2024**
(21) Application number: 22916133.6
(22) Date of filing: 27.12.2022
(51) Int. Cl.: A61K 36/28, A23L 33/105

(54) **METHOD FOR PRODUCING EXTRACT COMPOSITION**

(30) Priority: 28.12.2021 JP 2021214512
(71) Applicant: Kao Corporation, Tokyo 103-8210 (JP)
(72) Inventor: YAMAZAWA Yuka, Wakayama-shi, Wakayama 640-8580 (JP); DOI Haruhiko, Wakayama-shi, Wakayama 640-8580 (JP); KAYAKUBO Daisuke, Wakayama-shi, Wakayama 640-8580 (JP)
(74) Representative: Hoffmann Eitle
(86) International application number: PCT/JP2022/048344
(87) International publication number: WO 2023/127911

(57) **Abstract**

In one aspect, provided is a method for producing an extract composition with which a concentration ratio (a saccharide/an aglycone) between the saccharide (e.g., glucose) and the aglycone having a polyphenol structure (e.g., apigenin) in an extract of a harvested herbaceous plant containing a polyphenol can be reduced, and a concentration ratio (a glycoside/an aglycone) between the glycoside having a polyphenol structure (e.g., chamaemeloside) and its aglycone (e.g., apigenin) can be reduced without treatment using yeast, treatment using an organic acid under high temperature and high pressure, enzyme inactivation treatment at a high temperature that is required when enzyme is added, or the like. In one aspect, the present disclosure relates to a method for producing an extract composition, including steps (1) and (2) below
(1) The step of bringing a harvested herbaceous plant into contact with water or an aqueous surfactant solution at a temperature of 10°C or more and 95°C or less for 1 hour or more.
(2) The step of obtaining an extract from the herbaceous plant after the step (1).

## Description

### Technical Field

The present disclosure relates to a method for producing an extract composition, a method for producing a topical product or a food product, an extract composition, and a topical product or a food product.

### Background Art

Apigenin is a flavonoid (one type of polyphenol) contained in plants such as German chamomile (*Matricaria recutita L.*), Roman chamomile (*Anthemis nobilis L*)*,* dahlia (*Dahlia pinnata*), Chinese daphne (*Daphne genkwa*), and sorghums (*Sorghum nervosum Bess*), and is known to be useful as a component of topical products such as cosmetics, pharmaceuticals, and quasi-drugs because apigenin has urease activity inhibitory effects, antioxidant effects, melanin production promoting effects, and the like.

Also, a polyphenol includes a glycoside, in which a saccharide such as glucose is bound, and an aglycone, in which a saccharide is released from a glycoside and a glycosyl group is replaced with a hydrogen atom. In general, aglycones are known to have many advantages over glycosides, such as higher antioxidant power and higher permeability into the body.

However, plant extracts usually contain saccharides such as glucose, and it has been reported that such saccharides impair the biological activity of the active ingredients in the extracts. Therefore, various methods for reducing the amount of saccharides in plant extracts have been studied.

For example, JP 2020-193197A (Patent Document 1) proposes a method for preparing a plant extract that is rich in polyphenols and/or substantially free of monosaccharides, the method including a step of fermenting a plant extract using yeast belonging to the variety *Saccharomyces cerevisiae var. bayanus.*

JP 2018-148852A (Patent Document 2) proposes a method for producing a turmeric extract from which carbohydrates are removed while useful components contained in turmeric are left due to turmeric being hydrolyzed using an organic acid. Examples of the same patent document disclose that it is possible to obtain a turmeric extract from which carbohydrates have been removed by hydrolyzing turmeric starch using an aqueous solution of citric acid under high temperature and high pressure.

Meanwhile, WO 2019/044474 (Patent Document 3) discloses a method for producing an aglycone from a glycoside using a glycoside degrading enzyme.

### Disclosure of Invention

In one aspect, the present disclosure relates to a method for producing an extract composition, including steps (1) and (2) below
(1) The step of bringing a harvested herbaceous plant into contact with water or an aqueous surfactant solution at a temperature of 10°C or more and 95°C or less for 1 hour or more.
(2) The step of obtaining an extract from the herbaceous plant after the step (1).

In one aspect, the present disclosure relates to a method for producing a topical product or a food product, the method including a step of producing an extract composition using the method for producing an extract composition according to the present disclosure.

In one aspect, the present disclosure relates to an extract composition, in which a concentration ratio between a saccharide and an aglycone having a polyphenol structure (the saccharide/the aglycone) is 15 or less, a concentration ratio between a glycoside having a polyphenol structure and its aglycone (the glycoside/the aglycone) is 1.5 or less, and an aglycone conversion rate obtained using Formula (I) below is 40% or more. Aglycone conversion rate (%) = aglycone concentration/(glycoside concentration + aglycone concentration)×100

In one aspect, the present disclosure relates to a topical product or a food product containing the extract composition according to the present disclosure or a purified product thereof.

### Description of the Invention

As described above, various methods for reducing the amount of saccharide in plant extracts have been studied. However, yeast is used in the production method proposed in Patent Document 1, and thus fermentation takes 24 hours or more, and air bubbles need to be removed by stabilizing the fermented extract overnight. The production method proposed in Patent Document 2 requires hydrolysis using an organic acid under high temperature and high pressure.

Also, various methods for reducing the amount of glycosides in plant extracts and increasing the amount of aglycones have been studied. However, in Patent Document 3, a glycoside degrading enzyme needs to be separately prepared, and furthermore, after the glycoside degrading enzyme has acted, enzyme inactivation treatment needs to be performed at a high temperature.

In view of this, the present disclosure provides an extract composition with which a concentration ratio (a saccharide/an aglycone) between the saccharide (e.g., glucose) and the aglycone having a polyphenol structure (e.g., apigenin) in an extract of a harvested herbaceous plant containing a polyphenol can be reduced, and a concentration ratio (a glycoside/an aglycone) between the glycoside having a polyphenol structure (e.g., chamaemeloside, etc.) and its aglycone (e.g., apigenin) can be reduced without treatment using yeast, treatment using an organic acid under high temperature and high pressure, enzyme inactivation treatment at a high temperature that is required when enzyme is added, or the like, a method for producing the same, a method for producing a topical product or a food product using the same, and a topical product or a food product.

In one aspect, the present disclosure provides a method for producing an extract composition with which a concentration ratio (a saccharide/an aglycone) between the saccharide (e.g., glucose) and the aglycone having a polyphenol structure (e.g., apigenin) in an extract of a harvested herbaceous plant containing a polyphenol can be reduced, and a concentration ratio (a glycoside/an aglycone) between the glycoside having a polyphenol structure (e.g., chamaemeloside, etc.) and its aglycone (e.g., apigenin) can be reduced without treatment using yeast, treatment using an organic acid under high temperature and high pressure, enzyme inactivation treatment at a high temperature that is required when enzyme is added, or the like.

In the present disclosure, a polyphenol can include a glycoside and an aglycone. In the present disclosure, unless otherwise specified, the glycoside may be a glycoside having a polyphenol structure, and the aglycone may be an aglycone having a polyphenol structure.

The present disclosure is based on findings that, compared to a case where a harvested herbaceous plant is not immersed in water or an aqueous surfactant solution, the concentration of a saccharide such as glucose and the concentration of a glycoside such as chamaemeloside in the extract can be reduced by immersing a harvested herbaceous plant containing a polyphenol in water or an aqueous surfactant solution at a predetermined temperature for a predetermined time.

That is, in one aspect, the present disclosure relates to a method for producing an extract composition (also referred to as a "method for producing an extract composition according to the present disclosure"), the method including:
a step (1) of bringing a harvested herbaceous plant into contact with water or an aqueous surfactant solution at a temperature of 10°C or more and 95°C or less for 1 hour or more; and
a step (2) of obtaining an extract from the herbaceous plant after the step (1).

Although details of the mechanism of the expression of the effects of the present disclosure are unclear, the following is inferred.

It is conceivable that in the step (1), by bringing the harvested herbaceous plant into contact with water or an aqueous surfactant solution at a predetermined temperature, highly hydrophilic saccharides are washed and removed from plants, whereas highly hydrophobic aglycones such as apigenin remain in the plant body. It is also conceivable that hydrolysis of glycosides proceeds by glycolytic enzymes contained in the plant, and aglycones such as apigenin are produced. It is conceivable that, in the step (2), by extracting the aglycones such as apigenin, it is possible to reduce the concentration ratio (a saccharide/an aglycone) between the saccharide (e.g., glucose) and the aglycone (e.g., apigenin) in an extract of a herbaceous plant containing a polyphenol to a predetermined value or less, and reduce the concentration ratio (a glycoside/an aglycone) between the glycoside (e.g., chamaemeloside) and its aglycone (e.g., apigenin).

However, the present disclosure need not be construed as being limited to these mechanisms.

According to the present disclosure, in one or more embodiments, it is possible to reduce the concentration ratio (also referred to as the "concentration ratio (a saccharide/an aglycone)" hereinafter) between the saccharide (e.g., glucose) and the aglycone having a polyphenol structure (e.g., apigenin) in an extract of the harvested herbaceous plant containing a polyphenol, and reduce the concentration ratio (also referred to as the "concentration ratio (a glycoside/an aglycone)") between the glycoside having a polyphenol structure (e.g., chamaemeloside, etc.) and its aglycone (e.g., apigenin), without treatment using yeast, treatment using an organic acid under high temperature and high pressure, enzyme inactivation treatment at a high temperature that is required when enzyme is added, or the like. According to the present disclosure, in one or more embodiments, it is possible to obtain an extract having a high concentration of aglycones such as apigenin, and having a reduced concentration of saccharides such as glucose and a reduced concentration of glycosides such as chamaemeloside. It is possible to produce topical products or food products such as cosmetics, using such an extract.

### Step (1)

The step (1) in the method for producing an extract composition according to the present disclosure is a step of bringing a harvested herbaceous plant into contact with water or an aqueous surfactant solution at a temperature of 10°C or more and 95°C or less for 1 hour or more. Examples of a method for bringing a herbaceous plant into contact with water or an aqueous surfactant solution include immersion, spraying, and the like, and immersion is preferable. Therefore, in one or more embodiments, the step (1) is preferably a step of immersing the harvested herbaceous plant in water or the aqueous surfactant solution (also referred to as an "immersion solution" hereinafter) at a temperature of 10°C or more and 95°C or less for 1 hour or more.

In one or more embodiments, in the step (1), no enzyme is added to the contact between the herbaceous plant and water or the aqueous surfactant solution. In the present disclosure, the wording "no enzyme is added" indicates that an enzyme is not added separately from the herbaceous plant in one or more embodiments. This can reduce the concentration ratio (the saccharide/the aglycone) in the extract of the harvested herbaceous plant containing a polyphenol and reduce the concentration ratio (the glycoside/the aglycone), without requiring enzyme inactivation treatment at a high temperature that is required when enzyme is added.

Herbaceous plants are plants whose secondary tissues do not become lignified and do not grow thickly, and are classified into annuals, biennials, and perennials. As for annual herbaceous plants, the entire plant including the underground parts flowers and bears fruit within one year after germination, and then dies. Biennial herbaceous plants grow exclusively vegetatively in the first year, and flower and fruit in the second year. In contrast, plants of which at least underground parts survive for two or more years refer to perennial herbaceous plants. In the present disclosure, examples of herbaceous plants include, in one or more embodiments, herbaceous plants containing polyphenols, and include, in one or more other embodiments, herbaceous plants containing components to be extracted.

There is no particular limitation on types of herbaceous plants containing polyphenols as long as they contain aglycones having a polyphenol structure, and from the viewpoint of containing a large amount of vegetable flavonoids, examples thereof include plants of the Asteraceae, plants of the Umbelliferae, plants of the Lamiaceae, plants of the Hypericaceae, and plants of the Faboideae. From the viewpoint of efficiently extracting apigenin, plants of the Asteraceae are preferably spray mum, German chamomile (Japanese name: Camitsure), Roman chamomile (Japanese name: Roman Camitsure), dandelions, sunflower, and marigold, or related species thereof, and Roman chamomile is more preferable. Plants of the Umbelliferae include ashitaba, celery, dill, and parsley. Plants of the Lamiaceae include perilla, basil, rosemary, lavender, sage, oregano, thyme, red perilla, peppermint, and lemon balm. Plants of the Hypericaceae include *Hypericum perforatum.* Plants of the Faboideae include rooibos. In particular, from the viewpoint of efficiently extracting apigenin, the herbaceous plant is preferably at least one selected from German chamomile, Roman chamomile, parsley, perilla, oregano, and rooibos.

In the present disclosure, a polyphenol refers to, in one or more embodiments, a compound having two or more phenolic hydroxyl groups (a hydroxyl group bonded to an aromatic ring such as a benzene ring or a naphthalene ring) in the same molecule, and in one or more embodiments, a flavonoid compound such as flavones.

In the present disclosure, in one or more embodiments, the aglycone is at least one selected from apigenin, daidzein, quercetin, and naringenin. The aglycone includes, in one or more embodiments, apigenin. The aglycone is, in one or more embodiments, apigenin.

The saccharide is, in one or more embodiments, at least one selected from glucose, apiose, fructose, maltose, and lactose.

The glycoside is, in one or more embodiments, at least one selected from apigenin-7-glucoside, chamaemeloside, apiin, daidzin, quercitrin, rutin, and naringin.

In the step (1), the harvested herbaceous plant refers to, in one or more embodiments, the entirety or part of the herbaceous plant. Examples of the part of the herbaceous plant include an above-ground part, and examples of the above-ground part include flowers, petals, buds, leaves, and stems. For example, when an herbaceous plant containing apigenin is a spray mum, German chamomile, or Roman chamomile, from the viewpoint of efficiently extracting apigenin, the herbaceous plant is preferably a flower, petal, or bud, and more preferably a flower.

From the viewpoint of efficiently extracting an aglycone such as apigenin, the herbaceous plant in the step (1) was harvested preferably within the past 7 days, more preferably within the past 3 days, and further preferably within the past 1 day. Also, from the same viewpoint, as for the herbaceous plant in the step (1), preferably 30 minutes or more, more preferably 1 hour or more, and further preferably 12 hours or more have elapsed since the herbaceous plant was harvested.

In one or more embodiments, the herbaceous plant in the step (1) may be dried or frozen after the plant is harvested, and then preserved. In the present disclosure, preservation includes allowing to stand, leaving, or storage. Examples of the drying method include drying in the sun, drying in the shade, and drying using a dryer. Examples of the freezing method include freezing in a freezer at -5°C or less, or freezing using liquid nitrogen.

In the step (1), from the viewpoint of efficiently extracting an aglycone such as apigenin, from the viewpoint of reducing a saccharide concentration, and from the viewpoint of reducing the concentration ratio (the saccharide/the aglycone) and the concentration ratio (the glycoside/the aglycone), a period of time during which the harvested herbaceous plant is in contact with water or an aqueous surfactant solution is 1 hour or more, preferably 2 hours or more, more preferably 6 hours or more, even more preferably 12 hours or more, and further preferably 24 hours or more, and from the viewpoint of workability, preferably 168 hours or less, and more preferably 96 hours or less.

In the step (1), from the viewpoint of efficiently extracting a polyphenol such as apigenin, from the viewpoint of reducing the saccharide concentration, and from the viewpoint of reducing the concentration ratio (the saccharide/the aglycone) and the concentration ratio (the glycoside/the aglycone), the temperature of water or an aqueous surfactant solution used for the contact is 10°C or more, preferably 20°C or more, more preferably 40°C or more, and even more preferably 45°C or more, and is 95°C or less, preferably less than 80°C, more preferably less than 70°C, even more preferably 65°C or less, and further preferably 60°C or less.

In the step (1), there is no particular limitation on the amount of water or the aqueous surfactant solution used for the contact, as long as water or the aqueous surfactant solution can be sufficiently in contact with the herbaceous plant. For example, from the viewpoint of efficiently extracting an aglycone such as apigenin, from the viewpoint of reducing the saccharide concentration, and from the viewpoint of reducing the concentration ratio (the saccharide/the aglycone) and the concentration ratio (the glycoside/the aglycone), the amount of an immersion solution (water or the aqueous surfactant solution) into which the herbaceous plant is immersed is preferably 2 times or more, more preferably 10 times or more, and even more preferably 20 times or more, and from the viewpoint of workability, is preferably 80 times or less, and more preferably 60 times or less, the wet weight of the herbaceous plant to be immersed. In the present disclosure, the "wet weight" includes the weight of a harvested herbaceous plant, the weight of an herbaceous plant that has not been completely dried, the weight of an herbaceous plant that is not subjected to drying treatment after harvesting, or the weight of a herbaceous plant containing moisture.

In the step (1), examples of the water used for the contact or the water used for preparing the aqueous surfactant solution include ultrapure water, pure water, ion-exchanged water, and distilled water.

Examples of the surfactant contained in the aqueous surfactant solution include surfactants such as nonionic surfactants, anionic surfactants, and cationic surfactants. In particular, from the viewpoint of efficiently extracting an aglycone such as apigenin, from the viewpoint of reducing the saccharide concentration, and from the viewpoint of reducing the concentration ratio (the saccharide/the aglycone) and the concentration ratio (the glycoside/the aglycone), nonionic surfactants are preferable. Examples of the nonionic surfactants include polyoxyethylene alkyl ethers, polyoxyalkylene derivatives, sorbitan fatty acid esters, polyoxyethylene sorbitan fatty acid esters, polyoxyethylene fatty acid esters, and polyoxyethylene hydrogenated castor oil. In particular, a nonionic surfactant having a polyoxyethylene chain having an average number of added moles of 10 or more and 100 or less is preferable. These surfactants may be used alone or in combination of two or more.

From the viewpoint of efficiently extracting an aglycone such as apigenin, from the viewpoint of reducing the saccharide concentration, and from the viewpoint of reducing the concentration ratio (the saccharide/the aglycone) and the concentration ratio (the glycoside/the aglycone), the content of a surfactant in the aqueous surfactant solution is preferably 10 ppm or more, more preferably 50 ppm or more, and even more preferably 100 ppm or more, and from the same viewpoint, is preferably 10,000 ppm or less, more preferably 5,000 ppm or less, and even more preferably 1,000 ppm or less. When surfactants are used in a combination of two or more, the content of the surfactants in the aqueous surfactant solution refers to the total content thereof. Note that 1% by mass is 10,000 ppm (the same applies to the following) in the present disclosure.

In one or more embodiments, the water or the aqueous surfactant solution used for the contact may further contain other components as needed. Examples of the other components include a moisturizing agent for preventing dryness, an antiseptic agent for preventing rot, and a spreading agent for facilitating permeation to the plant body.

From the viewpoint of workability, in one or more embodiments, it is preferable that the water or the aqueous surfactant solution used for the contact does not contain organic acids, citric acid, and tartaric acid.

From the viewpoint of preservation stability, in one or more embodiments, it is preferable that the water or the aqueous surfactant solution used for the contact contains organic acids, citric acid, and tartaric acid.

In one or more embodiments, from the viewpoint of efficiently extracting an aglycone such as apigenin, the step (1) preferably includes a step of drying or freezing the harvested herbaceous plant before the contact.

When the harvested herbaceous plant is to be dried in the step of drying or freezing before the contact, from the viewpoint of efficiently extracting an aglycone such as apigenin, the drying temperature is preferably 10°C or more, more preferably 20°C or more, and even more preferably 35°C or more, and is preferably 100°C or less, more preferably 80°C or less, and even more preferably 45°C or less. From the same viewpoint, the drying time is preferably 3 hours or more, more preferably 6 hours or more, and even more preferably 24 hours or more, and is preferably 96 hours or less, more preferably 72 hours or less, and even more preferably 48 hours or less. Examples of the drying method before the contact include drying in the sun, drying in the shade, and drying using a dryer. Here, as an indicator of the dry state of an herbaceous plant, for example, when the weight change during 24 hours in a drying step is within 3%, it can be determined that the plant is in the dry state.

When the harvested herbaceous plant is to be frozen in the step of drying or freezing before the contact, from the viewpoint of efficiently extracting aglycones such as apigenin, the freezing temperature is preferably -196°C or more, more preferably -40°C or more, and even more preferably -20°C or more, and is preferably 0°C or less, more preferably -1°C or less, and even more preferably -5°C or less. More specifically, the freezing temperature is preferably -196°C or more and 0°C or less, more preferably -40°C or more and -1°C or less, and even more preferably -20°C or more and -5°C or less. From the same viewpoint, the freezing time is preferably 3 hours or more, more preferably 6 hours or more, and even more preferably 12 hours or more, and from the viewpoint of workability, is preferably within 72 hours, and more preferably within 48 hours. Examples of the freezing method before the contact include freezing using a freezer at -5°C or less, or freezing using liquid nitrogen.

In one or more embodiments, from the viewpoint of efficiently extracting an aglycone such as apigenin, the step (1) preferably includes a step of drying the harvested herbaceous plant after the contact.

In the step of drying after the contact, from the viewpoint of efficiently extracting an aglycone such as apigenin, the drying temperature is preferably 10°C or more, more preferably 20°C or more, and even more preferably 40°C or more, and is preferably 100°C or less, more preferably 80°C or less, and even more preferably 60°C or less. From the same viewpoint, the drying time is preferably 6 hours or more, more preferably 12 hours or more, and even more preferably 24 hours or more, and is preferably 96 hours or less, more preferably 72 hours or less, and even more preferably 48 hours or less.

Examples of the drying method after the contact include drying in the sun, drying in the shade, and drying using a dryer.

From the viewpoint of efficiently extracting an aglycone such as apigenin, the step (1) may further include a step of crushing the herbaceous plant after being dried (after the step of drying after the contact). Examples of the crushing method include crushing using a bead mill, a cutter mill, or the like.

### Step (2)

The step (2) in the method for producing an extract composition according to the present disclosure is a step of obtaining an extract from the herbaceous plant after the step (1). Examples of a method for extracting an extract include a method in which the extract is eluted from the herbaceous plant after the step (1) by bringing the herbaceous plant into contact with an extraction solvent. Examples of a method for contacting the extraction solvent include a method for immersing the herbaceous plant into the extraction solvent. In one or more embodiments, the extract can be obtained through filtration using a filter. In one or more embodiments, the step (2) is a step of obtaining the extract by immersing, in an extraction solvent, the herbaceous plant after the step (1), and filtering the resulting mixture using a filter.

An extraction target component in the extract composition according to the present disclosure, that is, a component of the extract composition obtained in the step (2) is, for example, an aglycone having a polyphenol structure, such as apigenin. Examples of aglycones include those mentioned above.

It is possible to use both polar solvents and non-polar solvents as a solvent for extracting an aglycone such as apigenin. Examples of the extraction solvent include water; alcohols such as methanol, ethanol, propanol, and butanol; polyhydric alcohols such as 1,3-propanediol, dipropylene glycol, pentanediol, hexanediol, propylene glycol, and butylene glycol; ketones such as acetone and methyl ethyl ketone; esters such as methyl acetate and ethyl acetate; linear and cyclic ethers such as tetrahydrofuran and diethyl ether; polyethers such as polyethylene glycol; hydrocarbons such as squalane, hexane, cyclohexane, and petroleum ether; esters of fatty acids and polyhydric alcohols, such as caprylic/capric triglyceride; aromatic hydrocarbons such as toluene; halogenated hydrocarbons such as dichloromethane, chloroform, and dichloroethane; and carbon dioxide. In particular, from the viewpoint of efficiently extracting an aglycone such as apigenin, an aqueous solution of butylene glycol is preferable. The concentration of the aqueous butylene glycol solution is preferably 20% by mass to 90% by mass.

From the viewpoint of efficiently extracting an aglycone such as apigenin, the amount of the extraction solvent used for extracting the aglycone such as apigenin is preferably 3 times or more, more preferably 5 times or more, and even more preferably 10 times or more, and from the same viewpoint, is preferably 100 times or less, more preferably 80 times or less, and even more preferably 60 times or less, the weight of the herbaceous plant after the step (1) or the dry weight thereof.

From the viewpoint of efficiently extracting an aglycone such as apigenin, a period of time during which the extraction solvent for extracting the aglycone such as apigenin is in contact with a flower and/or a leaf, or the immersion time during which a flower and/or a leaf is immersed in the extraction solvent, is preferably 5 hours or more, more preferably 12 hours or more, and even more preferably 24 hours or more, and from the same viewpoint, is preferably 240 hours or less, more preferably 200 hours or less, and even more preferably 160 hours or less.

From the viewpoint of efficiently extracting an aglycone such as apigenin, the temperature of the extraction solvent that is brought into contact with the flower and/or the leaf, or the immersion temperature at which the flower and/or the leaf is immersed in the extraction solvent, is preferably 5°C or more, more preferably 20°C or more, and even more preferably 35°C or more, and from the same viewpoint, is preferably 80°C or less, more preferably 60°C or less, even more preferably 50°C or less, and further preferably 40°C or less.

Examples of filters used for filtration include filter paper, membrane filters, cartridge filters, and disposable filters.

The extract composition produced using the method for producing an extract composition according to the present disclosure may be further purified to obtain a purified product. Therefore, in one or more embodiments, the step (2) may have a step of purifying the extract. In one or more other embodiments, the method for producing an extract composition according to the present disclosure may further include a step of purifying the extract after the step (2).

### Extract composition

In one aspect, the present disclosure relates to an extract composition produced using the method for producing an extract composition according to the present disclosure (also referred to as an "extraction composition of the present disclosure" hereinafter). In one or more embodiments, the extract composition of the present disclosure is an extract composition, in which a concentration ratio between a saccharide and an aglycone having a polyphenol structure (the saccharide/the aglycone) is 15 or less, a concentration ratio between a glycoside having a polyphenol structure and its aglycone (the glycoside/the aglycone) is 1.5 or less, and an aglycone conversion rate represented by Formula (I) below is 40% or more. According to the present disclosure, by using the method for producing an extract composition according to the present disclosure, it is possible to obtain, from a harvested herbaceous plant, an extract composition having a high concentration of aglycone such as apigenin, a low saccharide concentration, and a low glycoside concentration.

Here, the concentration of aglycone in the extract composition of the present disclosure is preferably 200 ppm or more, more preferably 300 ppm or more, and even more preferably 400 ppm or more, and is preferably 10000 ppm or less, and more preferably 2000 ppm or less. The aglycone concentration is, in one or more embodiments, the concentration of aglycones including at least one selected from apigenin, daidzein, quercetin, and naringenin, and in one or more other embodiments, the concentration of aglycones including apigenin, and in one or more other embodiments, the concentration of apigenin. In the present disclosure, the aglycone concentration can be measured using the method described in Examples.

The concentration of saccharides in the extract composition of the present disclosure is preferably 2000 ppm or less, more preferably 1000 ppm or less, and even more preferably 500 ppm or less. In one or more embodiments, the saccharides are saccharides (a mixture of saccharides including glucose) that exhibit the maximum peak at an elution time of 1.4 minutes when the saccharides are analyzed through high-performance liquid chromatography (HPLC) under the following analysis conditions.

The wording "concentration of saccharides" in the present disclosure may be the concentration of glucose in one or more embodiments, the total concentration of glucose and fructose in one or more other embodiments, and the total concentration of two, three, or four types selected from glucose, apiose, fructose, maltose, and lactose in one or more other embodiments.

The concentration of saccharides can be measured using the method described in Examples.

### Analysis Conditions

Measurement Apparatus: Ultimate 3000 (HPLC), Corona ultra (detector) (manufactured by Dionex Corporation)
Column: InertSustain ODS-3 5 um 3.0×150 mm (manufactured by GL Sciences Inc.)
Eluent: formic acid 0.1% (solution A) and acetonitrile (solution B)
Gradient conditions: gradient from 95 (solution A): 5 (solution B) to 10 (solution A) : 90 (solution B)
Flow rate: 0.5 mL/min
Column temperature: 40°C
Detector: CAD

The glycoside concentration in the extract composition of the present disclosure is preferably 500 ppm or less, more preferably 300 ppm or less, and even more preferably 100 ppm or less. The glycoside concentration is, in one or more embodiments, the concentration of glycosides including at least one selected from apigenin-7-glucoside, chamaemeloside, apiin, daidzin, quercitrin, rutin, and naringin, and in one or more other embodiments, the concentration of apigenin glycoside including at least one of apigenin-7-glucoside and chamaemeloside. In the present disclosure, the glycoside concentration can be measured using the method described in Examples.

The concentration ratio (the saccharide/the aglycone) in the extract composition of the present disclosure is preferably 15 or less, more preferably 8 or less, even more preferably 5 or less, and further preferably 1 or less, and is preferably 0.01 or more, and more preferably 0.1 or more.

The concentration ratio (the glycoside/the aglycone) in the extract composition of the present disclosure is preferably 2 or less, more preferably 1.5 or less, even more preferably 1 or less, and further preferably 0.2 or less, and is preferably 0.001 or more, and more preferably 0.01 or more.

From the viewpoint of improving extract effects, the aglycone conversion rate in the extract composition of the present disclosure is preferably 40% or more, more preferably 60% or more, and even more preferably 80% or more.

In the present disclosure, the aglycone conversion rate is a value obtained using Formula (I) below Aglycone conversion rate (%) = aglycone concentration/(glycoside concentration + aglycone concentration)×100

The aglycone conversion rate is, in one or more embodiments, apigenin conversion rate obtained using the following formula. Apigenin conversion rate (%) = apigenin concentration/(apigenin-7-glucoside concentration + chamaemeloside concentration + apigenin concentration)×100

The amount of aglycone such as apigenin in the extract composition is preferably 0.01% by mass or more, more preferably 0.03% by mass or more, and even more preferably 0.05% by mass or more, and is preferably 1% by mass or less, more preferably 0.3% by mass or less, and even more preferably 0.1% by mass or less.

The extract composition produced using the method for producing an extract composition according to the present disclosure or a purified product thereof can be favorably used as a topical product or a food product. Therefore, in one aspect, the present disclosure relates to a method for producing a topical product or a food product, the method including a step of producing an extract composition using the method for producing an extract composition according to the present disclosure. Also, in another aspect, the present disclosure relates to a topical product or a food product containing the extract composition of the present disclosure or a purified product thereof.

In the present disclosure, examples of the topical product include perfume impaired products such as cosmetics, hair cosmetics, pharmaceuticals, quasi-drugs, bath salts, and toothpaste. Examples of the form of the topical product include creams, liquid lotions, emulsion lotions, sprays, and skin lotions. Here, the content of the extract composition or a purified product thereof in a topical product or a food product is preferably 0.0001% or more, more preferably 0.01% or more, and even more preferably 1% or more, and is preferably 10% or less, and more preferably 5% or less.
<1> A method for producing an extract composition, including:
   a step (1) of bringing a harvested herbaceous plant into contact with water or an aqueous surfactant solution at a temperature of 10°C or more and 95°C or less for 1 hour or more; and
   a step (2) of obtaining an extract from the herbaceous plant after the step (1).
<2> The production method according to <1>, in which the herbaceous plant in the step (1) was harvested preferably within the past 7 days, more preferably within the past 3 days, and further preferably within the past 1 day, and preferably 30 minutes or more, more preferably 1 hour or more, and even more preferably 12 hours or more have elapsed since the herbaceous plant was harvested.
<3> The production method according to <1> or <2>, in which, in the step (1), a period of time during which the harvested herbaceous plant is in contact with water or the aqueous surfactant solution is 1 hour or more, preferably 2 hours or more, more preferably 6 hours or more, even more preferably 12 hours or more, and further preferably 24 hours or more, and is more preferably 168 hours or less, and more preferably 96 hours or less.
<4> The production method according to any one of <1> to <3>, in which, in the step (1), the temperature of water or the aqueous surfactant solution used for the contact is 10°C or more, preferably 20°C or more, more preferably 40°C or more, and even more preferably 45°C or more, and is 95°C or less, preferably less than 80°C, more preferably less than 70°C, even more preferably 65°C or less, and further preferably 60°C or less.
<5> The production method according to any one of < 1> to <4>, in which, in the step (1), the amount of water or the aqueous surfactant solution used for the contact is preferably 2 times or more, more preferably 10 times or more, and even more preferably 20 times or more, and is preferably 80 times or less, and more preferably 60 times or less, the wet weight of the herbaceous plant to be immersed.
<6> The production method according to any one of < 1> to <5>, in which, in the step (1), a surfactant contained in the aqueous surfactant solution is preferably at least one selected from polyoxyethylene alkyl ethers, polyoxyalkylene derivatives, sorbitan fatty acid esters, polyoxyethylene sorbitan fatty acid esters, polyoxyethylene fatty acid esters, and polyoxyethylene hydrogenated castor oil, a nonionic surfactant having a polyoxyethylene chain having an average number of added moles of 10 or more and 100 or less is preferable, and the surfactant may be used alone or in combination of two or more.
<7> The production method according to any one of < 1> to <6>, in which the content of a surfactant in the aqueous surfactant solution is preferably 10 ppm or more, more preferably 50 ppm or more, and even more preferably 100 ppm or more, and is preferably 10,000 ppm or less, more preferably 5,000 ppm or less, and even more preferably 1,000 ppm or less.
<8> The production method according to any one of < 1> to <7>, in which, in the step (1), no enzyme is added to the contact between the herbaceous plant and water or the aqueous surfactant solution.
<9> The production method according to any one of < 1> to <8>, in which the step (1) includes a step of drying or freezing the harvested herbaceous plant before the contact.
<10> The production method according to any one of < 1> to <9>, in which, when the harvested herbaceous plant is to be dried in a step of drying before the contact, a drying temperature is preferably 10°C or more, more preferably 20°C or more, and even more preferably 35°C or more, and is preferably 100°C or less, more preferably 80°C or less, and even more preferably 45°C or less.
<11> The production method according to any one of <1> to <10>, in which, in a step of drying before the contact, a drying time is preferably 3 hours or more, more preferably 6 hours or more, and even more preferably 24 hours or more, and is preferably 96 hours or less, more preferably 72 hours or less, and even more preferably 48 hours or less.
<12> The production method according to any one of < 1> to <11>, in which a step (1) includes a step of drying the harvested herbaceous plant after the contact.
<13> The production method according to any one of <1> to <12>, in which, in a step of drying after the contact, a drying temperature is preferably 10°C or more, more preferably 20°C or more, and even more preferably 40°C or more, and is preferably 100°C or less, more preferably 80°C or less, and even more preferably 60°C or less.
<14> The production method according to any one of <1> to <13>, in which, in a step of drying after the contact, a drying time is preferably 6 hours or more, more preferably 12 hours or more, and even more preferably 24 hours or more, and is preferably 96 hours or less, more preferably 72 hours or less, and even more preferably 48 hours or less.
<15> The production method according to any one of <1> to <14>, in which the step (1) further includes a step of crushing the dried herbaceous plant.
<16> The production method according to any one of <1> to <15>, in which a crushing method is crushing using a bead mill or a cutter mill.
<17> The production method according to any one of <1> to <14>, in which the step (2) is a step of obtaining the extract from the herbaceous plant after the step (1), and the step (2) is a step of obtaining the extract by immersing, in an extraction solvent, the herbaceous plant after the step (1), and filtering the resulting solution using a filter.
<18> The production method according to any one of <1> to <17>, in which an extraction solvent is at least one selected from water, methanol, ethanol, propanol, butanol, propylene glycol, butylene glycol, 1,3-propanediol, dipropylene glycol, pentanediol, hexanediol, acetone, methyl ethyl ketone, methyl acetate, ethyl acetate, tetrahydrofuran, diethyl ether, polyethylene glycol, squalane, hexane, cyclohexane, petroleum ether, toluene, dichloromethane, chloroform, dichloroethane, caprylic/capric triglyceride, and carbon dioxide.
<19> The production method according to any one of <1> to <18>, in which the amount of an extraction solvent used is preferably 3 times or more, more preferably 5 times or more, and even more preferably 10 times or more, and is preferably 100 times or less, more preferably 80 times or less, and even more preferably 60 times or less, the weight of the herbaceous plant after the step (1) or the dry weight of the herbaceous plant after the step (1).
<20> The production method according to any one of <1> to <19>, in which a period of time during which an extraction solvent is in contact with a flower and/or a leaf, or an immersion time during which the flower and/or the leaf is immersed in the extraction solvent, is preferably 5 hours or more, more preferably 12 hours or more, and even more preferably 24 hours or more, and is preferably 240 hours or less, more preferably 200 hours or less, and even more preferably 160 hours or less.
<21> The production method according to any one of <1> to <20>, in which the temperature of an extraction solvent that is brought into contact with a flower and/or a leaf, or an immersion temperature at which the flower and/or the leaf is immersed in the extraction solvent, is preferably 5°C or more, more preferably 20°C or more, and even more preferably 35°C or more, and is preferably 80°C or less, more preferably 60°C or less, even more preferably 50°C or less, and further preferably 40°C or less.
<22> The production method according to any one of <1> to <21>, in which the herbaceous plant in the step (1) is the entirety or a part of the herbaceous plant.
<23> The production method according to any one of <1> to <22>, in which the herbaceous plant is an herbaceous plant containing a polyphenol.
<24> The production method according to any one of <1> to <23>, in which the herbaceous plant is a plant of the Asteraceae.
<25> The production method according to any one of <1> to <24>, in which the herbaceous plant is at least one selected from German chamomile, Roman chamomile, parsley, perilla, oregano, and rooibos.
<26> A method for producing a topical product or a food product, the method including a step of producing an extract composition using the production method according to any one of <1> to <25>.
<27> An extract composition, in which a concentration ratio between a saccharide and an aglycone having a polyphenol structure (the saccharide/the aglycone) is 15 or less, and a concentration ratio between a glycoside having a polyphenol structure and its aglycone (the glycoside/the aglycone) is 1.5 or less, and
   an aglycone conversion rate obtained using Formula (I) below is 40% or more, aglycone conversion rate (%) = aglycone concentration/(glycoside concentration + aglycone concentration)×100
<28> The extract composition according to <27>, in which the aglycone is at least one selected from apigenin, daidzein, quercetin, and naringenin,
   the saccharide is at least one selected from glucose, apiose, fructose, maltose, and lactose, and
   the glycoside is at least one selected from apigenin-7-glucoside, chamaemeloside, apiin, daidzin, quercitrin, rutin, and naringin.
<29> The extract composition according to <27> or <28>, in which the aglycone is apigenin.
<30> A topical product or a food product containing the extract composition according to any one of <27> to <29> or a purified product thereof.

### Examples

Hereinafter, the present disclosure will be described in more detail by way of examples, but these examples are merely exemplary, and the present disclosure is not limited to these examples.

### 1. Method for producing extract composition (Examples 1 to 19, Comparative Examples 1 to 9)

### Examples 1 to 6

Extract compositions of Examples 1 to 6 were obtained from harvested plants of the Asteraceae by performing steps (1) and (2) below

### Harvest of Asteraceae plants (Roman chamomile)

Roman chamomile (Japanese name: Roman Camitsure) pot seedlings were planted and grown in a hydroponic cultivation device (the temperature was 25°C). A liquid fertilizer for hydroponic cultivation was diluted with water such that the electrical conductivity was 1.0 mS/cm. After Roman chamomile bloomed, only the flowers were harvested.

### Step (1): Pretreatment

The harvested fresh Roman chamomile flowers were subjected to pretreatments 2 to 4 described below before extraction in a step (2). The pretreatment 1 was performed only in Examples 7 and 8 described below.
Pretreatment 1: Not performed in Examples 1 to 6.
Pretreatment 2: Harvested fresh flowers were immersed in water (immersion solution) under conditions (temperature, time) shown in Table 1. The amount of water used for immersion was 20 times (20 g) the weight of fresh flowers (unit: 1 g).
Pretreatment 3: The flowers immersed in the pretreatment 2 were dried for 24 hours in a dryer set at a temperature of 40°C.
Pretreatment 4: The flowers dried in the pretreatment 3 were crushed using a multi-bead shocker (manufactured by Yasui Kiki Corporation).

### Step (2): Extraction

The flowers were immersed in an aqueous solution of 1,3-butylene glycol (having a concentration of 60% by mass) in an amount of 20 times (4 g) the dry weight (0.2 g) of the flowers after the step (1). The immersion temperature was 60°C, and the immersion time was 24 hours.

Then, an extract composition was obtained by filtering the aqueous solution of 1,3-butylene glycol in which the flowers were immersed, using a filter (a disposable filter manufactured by ADVANTEC, the pore size was 0.45 µm).

### Example 7

An extract composition of Example 7 was obtained in the same manner as in Example 1, except that the drying treatment was performed in the pretreatment 1. The pretreatment 1 was carried out as follows.

### Step (1): Pretreatment 1

Fresh Roman chamomile flowers harvested within the past 7 days were dried for 24 hours in a dryer set at a temperature of 40°C.

### Example 8

An extract composition of Example 8 was obtained in the same manner as in Example 1, except that freezing treatment was performed in the pretreatment 1. The pretreatment 1 was carried out as follows.

### Step (1): Pretreatment 1

Fresh Roman chamomile flowers harvested within the past 7 days were frozen by immersing the flowers for 24 hours in a constant temperature bath set at -20°C.

### Examples 9 and 10

Extract compositions of Examples 9 and 10 were obtained in the same manner as in Example 1, except that an aqueous surfactant solution was used as an immersion solution in the pretreatment 2, instead of water. The amount of the aqueous surfactant solution used for immersion was 20 times (20 g) the wet weight of the flowers (unit: 1 g).

The aqueous surfactant solution was prepared as follows.

### Preparation of aqueous surfactant solution

Aqueous surfactant solutions of Examples 9 and 10 were prepared by mixing surfactants shown in Table 1 with water. The content (effective amount, ppm) of each component in the aqueous surfactant solution was as shown in Table 1. The water content was the remainder excluding the surfactant.

The following surfactants were used.

### Example 9: Nonionic surfactant (polyoxyethylene hydrogenated castor oil, the average number of moles of oxyethylene groups added: 60, "EMANON CH-60(K)" manufactured by Kao Corporation, the concentration was 100% by mass)

### Example 10: Nonionic surfactant (polyoxyethylene sorbitan monooleate, the average number of moles of oxyethylene groups added: 20, "RHEODOL TW-O120V" manufactured by Kao Corporation, the concentration was 97.3% by mass)

### Comparative Example 1

An extract composition of Comparative Example 1 was obtained in the same manner as in Example 1, except that the pretreatments 1 and 2 were not carried out, and the fresh flowers harvested in the pretreatment 3 were dried for 24 hours in a dryer set at a temperature of 40°C.

### Examples 11 to 13

Extract compositions of Examples 11 to 13 were obtained in the same manner as in Example 1, except that temperature conditions in the pretreatment 2 were changed as shown in Table 2.

### Example 14

An extract composition of Example 14 was obtained in the same manner as in Example 1, except that the pretreatment 4 was not carried out, and the extraction conditions in the step (2) were changed to "the temperature of 80°C and the time of 5 hours".

### Examples 15 to 19

Extract compositions of Example 15 to 19 were obtained in the same manner as in Example 1, except that the plant type was changed as shown in Table 2, and the pretreatment 4 was not carried out.

Here, the plants of Examples 15 to 19 shown in Table 2 purchased from the following manufacturers were used.
Umbelliferae plant (parsley): Leaf Corp.
Lamiaceae plant (oregano): Soken Ltd.
Asteraceae plant (German chamomile): Soken Ltd.
Lamiaceae plant (perilla): Leaf Corp.
Faboideae plant (rooibos): Soken Ltd.

### Comparative Example 2

An extract composition of Comparative Example 2 was obtained in the same manner as in Comparative Example 1, except that the plant type was changed as shown in Table 2, and the pretreatment 3 was not carried out.

### Comparative Example 3

An extract composition of Comparative Example 3 was obtained in the same manner as in Example 1, except that the temperature in the pretreatment 2 was changed to 100°C.

### Comparative Example 4

An extract composition of Example 4 was obtained in the same manner as in Example 14, except that the pretreatment 2 was not carried out.

### Comparative Example 5

An extract composition of Comparative Example 5 was obtained in the same manner as in Example 15, except that the pretreatments 2 and 3 were not carried out.

### Comparative Example 6

An extract composition of Comparative Example 6 was obtained in the same manner as in Example 16, except that the pretreatments 2 and 3 were not carried out.

### Comparative Example 7

An extract composition of Comparative Example 7 was obtained in the same manner as in Example 17, except that the pretreatments 2 and 3 were not carried out.

### Comparative Example 8

An extract composition of Comparative Example 8 was obtained in the same manner as in Example 18, except that the pretreatments 2 and 3 were not carried out.

### Comparative Example 9

An extract composition of Comparative Example 9 was obtained in the same manner as in Example 19, except that the pretreatments 2 and 3 were not carried out.

### 2. Evaluation

### Evaluation of apigenin concentration

The apigenin concentration in the obtained extract composition was measured using high-performance liquid chromatography (HPLC) under the following conditions.

### Measurement Conditions

Apparatus: Ultimate 3000 (HPLC), Corona ultra (detector) (manufactured by Dionex Corporation)
Column: InertSustain ODS-3 5 um 3.0×150 mm (manufactured by GL Sciences Inc.)
Eluent: formic acid 0.1% (solution A) and acetonitrile (solution B)
Gradient conditions: gradient from 95 (solution A) : 5 (solution B) to 10 (solution A) : 90 (solution B)
Flow rate: 0.5 mL/min
Column temperature: 40°C
Detector: CAD

### Evaluation of saccharide concentration

The saccharide concentration in the obtained extract composition was measured using high-performance liquid chromatography (HPLC) under the following conditions. The saccharide concentration measured under the following conditions is the concentration of a mixture of saccharides including glucose.

### Measurement Conditions

Apparatus: Ultimate 3000 (HPLC), Corona ultra (detector) (manufactured by Dionex Corporation)
Column: InertSustain ODS-3 5 um 3.0×150 mm (manufactured by GL Sciences Inc.)
Eluent: formic acid 0.1% (solution A) and acetonitrile (solution B)
Gradient conditions: gradient from 95 (solution A) : 5 (solution B) to 10 (solution A) : 90 (solution B)
Flow rate: 0.5 mL/min
Column temperature: 40°C
Elution time: 1.4 minutes
Detector: CAD

### Evaluation of glycoside concentration

The glycoside concentration in the obtained extract composition was measured using high-performance liquid chromatography (HPLC) under the following conditions.

### Measurement Conditions

Apparatus: Ultimate 3000 (HPLC), Corona ultra (detector) (manufactured by Dionex Corporation)
Column: InertSustain ODS-3 5 um 3.0×150 mm (manufactured by GL Sciences Inc.)
Eluent: formic acid 0.1% (solution A) and acetonitrile (solution B)
Gradient conditions: gradient from 95 (solution A) : 5 (solution B) to 10 (solution A): 90 (solution B)
Flow rate: 0.5 mL/min
Column temperature: 40°C
Detector: CAD

### Evaluation of apigenin conversion rate

The apigenin conversion rate in the obtained extract composition is a value obtained using the following formula. Apigenin conversion rate (%) = apigenin concentration/(apigenin-7-glucoside concentration + chamaemeloside concentration + apigenin concentration)×100

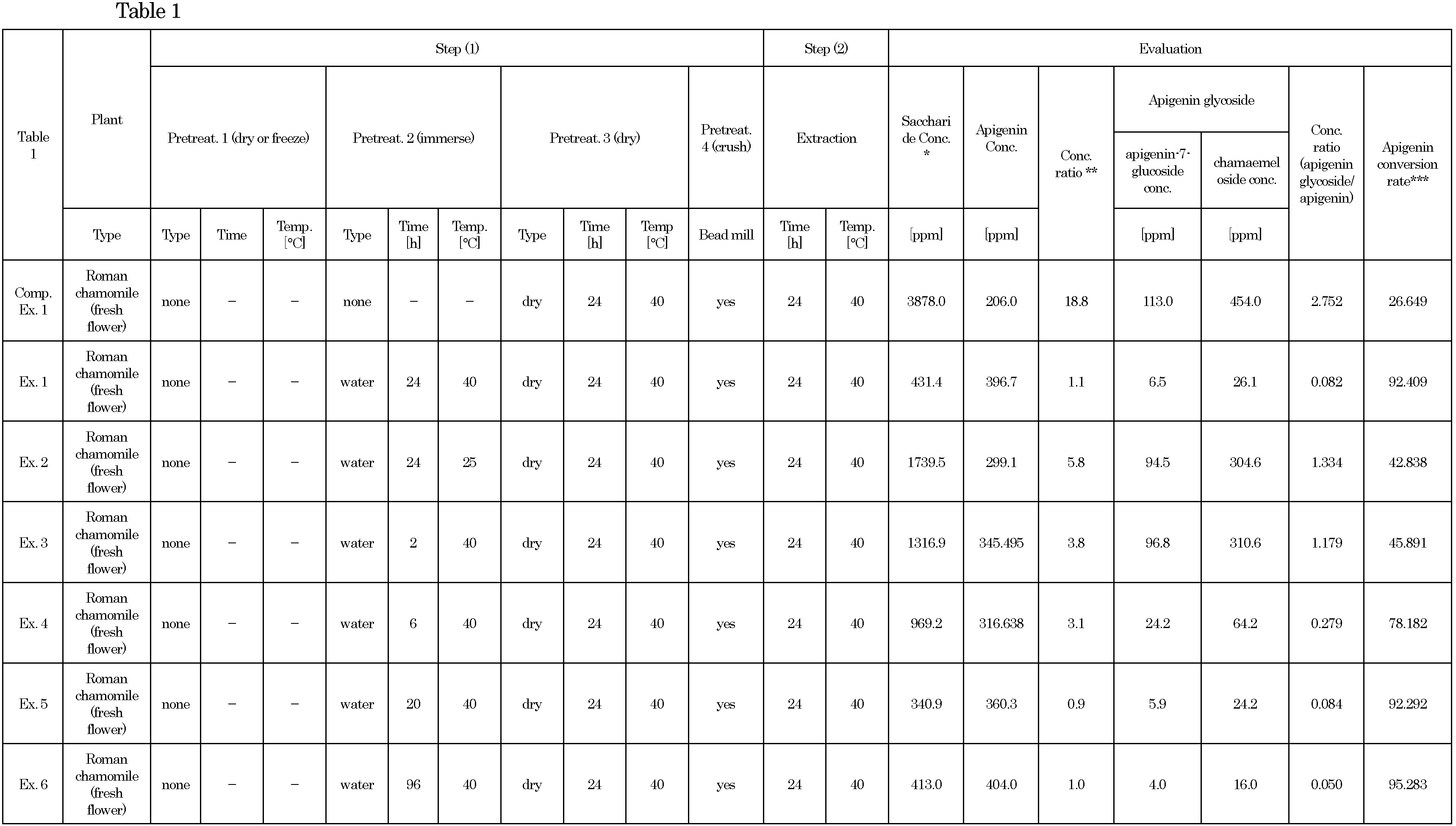

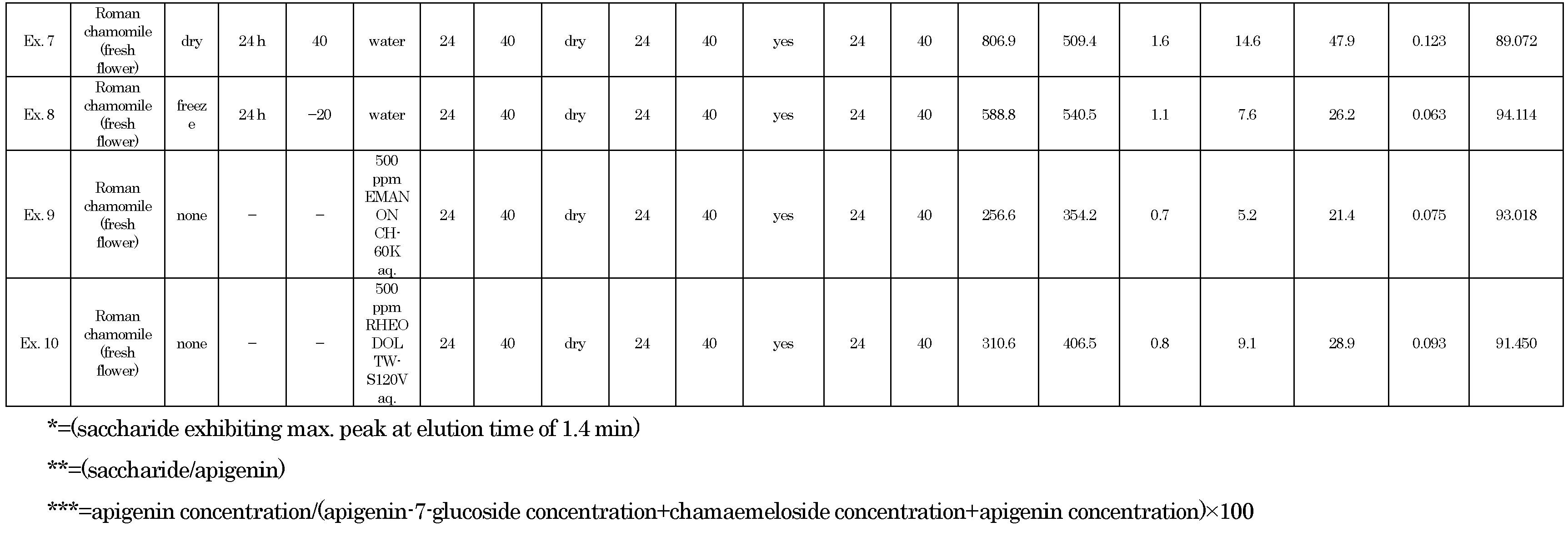

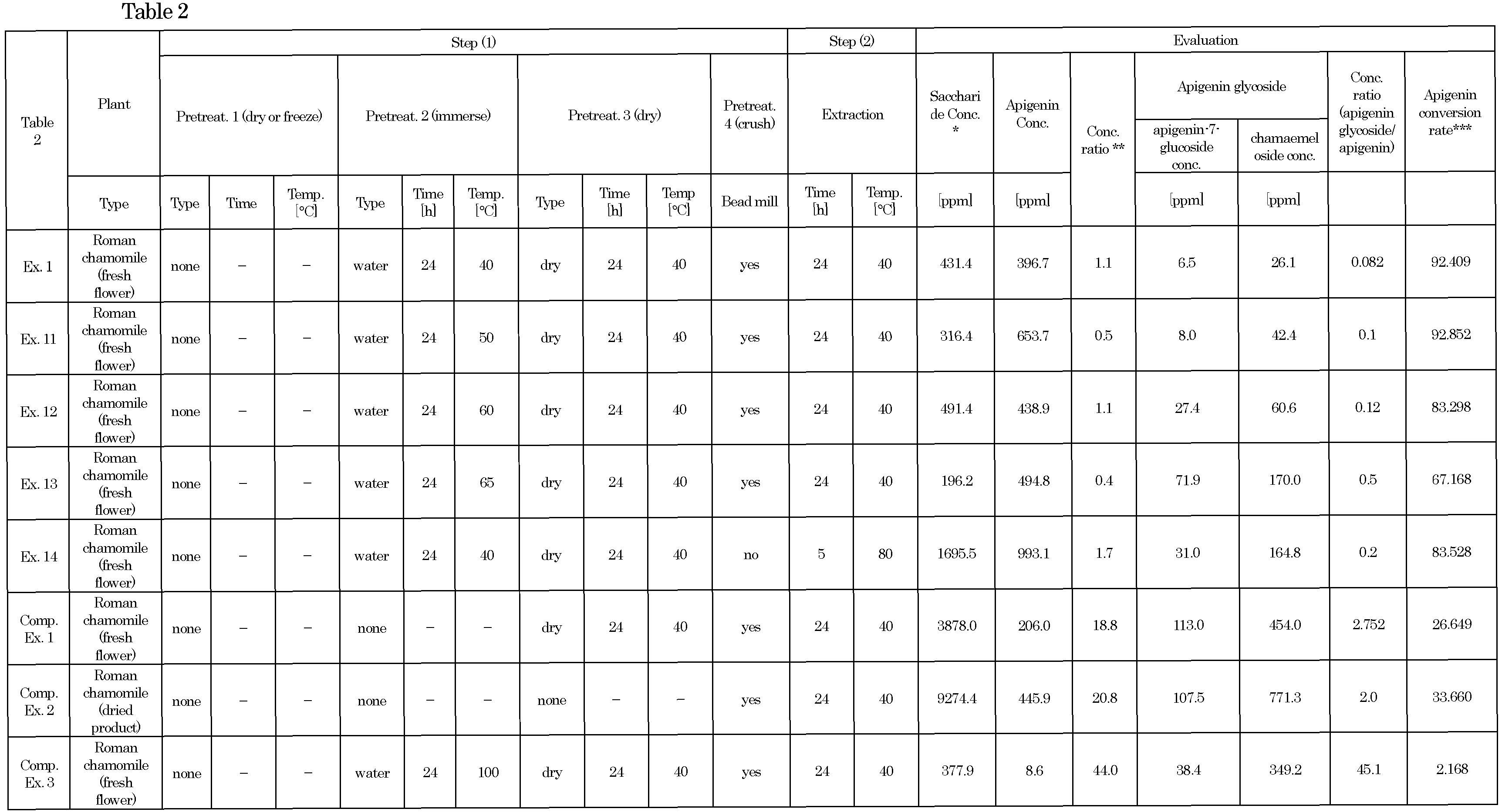

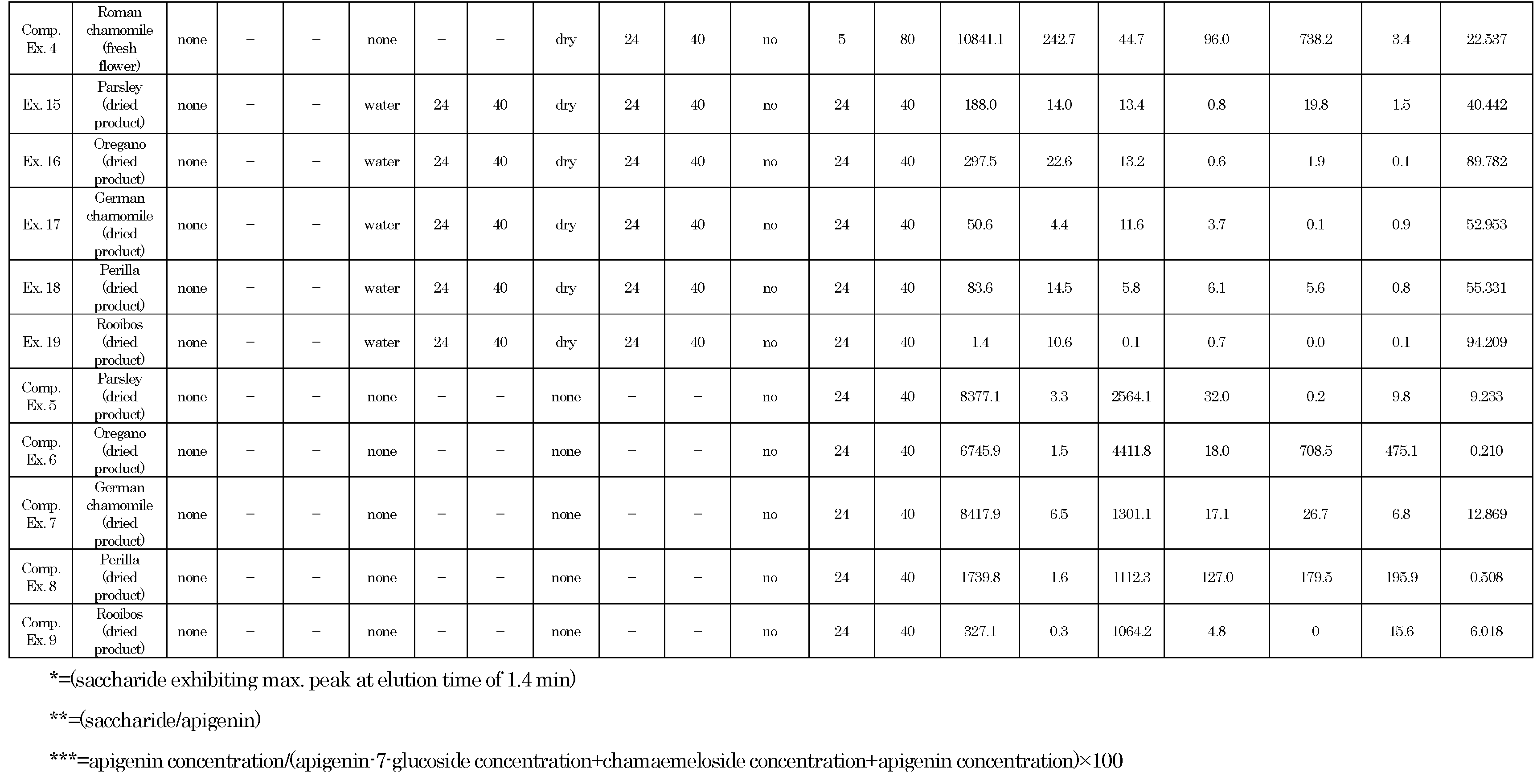

As shown in Table 1 above, it was found that, in Examples 1 to 10, in which at least the pretreatment 2 (immersion: 25°C to 40°C, 2 to 96 hours) was performed on the harvested Roman chamomile (Asteraceae plant), the saccharide and glycoside concentrations in the extract composition were reduced, the concentration ratio of the saccharide/the apigenin was reduced, and the concentration ratio of the glycoside/the apigenin was reduced, compared to Comparative Example 1, in which the pretreatment 2 was not performed. Furthermore, compared to Comparative Example 1, the apigenin conversion rate was improved in Examples 1 to 10.

As shown in Table 2 above, it was found that, in Examples 11 to 14, in which at least the pretreatment 2 (immersion: 40°C to 65°C, 24 hours) was performed on the harvested Roman chamomile (Asteraceae plant), the saccharide and glycoside concentrations in the extract composition were reduced, the concentration ratio of the saccharide/the apigenin was reduced, and the concentration ratio of the glycoside/the apigenin was reduced, compared to Comparative Examples 2 and 4, in which the pretreatment 2 was not performed, and Comparative Example 3, in which the temperature of the pretreatment 2 was 100°C. Furthermore, compared to Comparative Examples 2 to 4, the apigenin conversion rate was improved in Examples 11 to 14.

It was found that, in Examples 15 to 19, in which at least the pretreatment 2 (immersion: 40°C, 24 hours) was performed on the harvested parsley (Umbelliferae plant), oregano (Lamiaceae plant), German chamomile (Asteraceae plant), Perilla (Lamiaceae plant), and rooibos (Fabaceae plant), the saccharide and glycoside concentrations in the extract composition were reduced, the concentration ratio of the saccharide/the apigenin was reduced, and the concentration ratio of the glycoside/the apigenin was reduced, compared to Comparative Examples 5 to 9, in which the pretreatment 2 was not performed. Furthermore, compared to Comparative Examples 5 to 9, the apigenin conversion rate was improved in Examples 15 to 19.

Here, details of the saccharides in Example 11 were analyzed and shown in Table 3.

Details of saccharides were measured using high-performance liquid chromatography (HPLC) under the following conditions, and analysis results (unit: ppm) were shown in Table 3.

### Measurement Conditions

Apparatus: Shimazu Nexcera (HPLC), AB sciex Triple (detector) (manufactured by Shimazu Corporation)
Column: Shodex HILICpak VG-50 2D 5 um 2.1×150 mm (manufactured by Showa Denko K.K.)
Eluent: 0.2% ammonium water (solution A) and acetonitrile (solution B)
Gradient conditions: gradient from 5 (solution A) : 95 (solution B) to 50 (solution A) : 50 (solution B)
Flow rate: 0.3 mL/min
Column temperature: 40°C
Detector: MS/MS

**Table 3**

| Table 3 | Detailed analysis of saccharides of Ex. 11 (unit: ppm) |
|---|---|
| Glucose | 23 |
| Fructose | 48 |
| Sucrose | 0 |

Based on analysis results shown in Table 3, it is found that the saccharides in Example 11 include glucose and fructose.

### Industrial Applicability

According to the present disclosure, it is possible to provide a method for producing an extract composition that can efficiently reduce the concentration ratio between a saccharide and an aglycone having a polyphenol structure (the saccharide/the aglycone), and the concentration ratio between a glycoside having a polyphenol structure and its aglycone.

## Claims

1. A method for producing an extract composition, comprising:
a step (1) of bringing a harvested herbaceous plant into contact with water or an aqueous surfactant solution at a temperature of 10°C or more and 95°C or less for 1 hour or more; and
a step (2) of obtaining an extract from the herbaceous plant after the step (1).

2. The production method according to claim 1,
wherein the step (1) includes a step of drying or freezing the harvested herbaceous plant before the contact.

3. The production method according to claim 1 or 2,
wherein the step (1) includes a step of drying the harvested herbaceous plant after the contact.

4. The production method according to claim 3,
wherein the step (1) further includes a step of crushing the dried herbaceous plant.

5. The production method according to any one of claims 1 to 4,
wherein a surfactant contained in the aqueous surfactant solution used in the step (1) is a nonionic surfactant.

6. The production method according to any one of claims 1 to 5,
wherein the temperature of water or the aqueous surfactant solution used in the step (1) is 20°C or more and 65°C or less.

7. The production method according to any one of claims 1 to 6,
wherein, in the step (1), no enzyme is added to the contact between the herbaceous plant and water or the aqueous surfactant solution.

8. The production method according to any one of claims 1 to 7,
wherein the herbaceous plant in the step (1) is the entirety or a part of the herbaceous plant.

9. The production method according to any one of claims 1 to 8,
wherein the herbaceous plant is a herbaceous plant containing a polyphenol.

10. The production method according to any one of claims 1 to 9,
wherein the herbaceous plant is a plant of the Asteraceae.

11. The production method according to any one of claims 1 to 9,
wherein the herbaceous plant is at least one selected from German chamomile, Roman chamomile, parsley, perilla, oregano, and rooibos.

12. A method for producing a topical product or a food product, comprising:
a step of producing an extract composition using the production method according to any one of claims 1 to 11.

13. An extract composition,
wherein a concentration ratio between a saccharide and an aglycone having a polyphenol structure (the saccharide/the aglycone) is 15 or less, and a concentration ratio between a glycoside having a polyphenol structure and its aglycone (the glycoside/the aglycone) is 1.5 or less, and
an aglycone conversion rate obtained using Formula (I) below is 40% or more, aglycone conversion rate (%) = aglycone concentration/(glycoside concentration + aglycone concentration)×100 (I).

14. The extract composition according to claim 13,
wherein the aglycone is at least one selected from apigenin, daidzein, quercetin, and naringenin,
the saccharide is at least one selected from glucose, apiose, fructose, maltose, and lactose, and
the glycoside is at least one selected from apigenin-7-glucoside, chamaemeloside, apiin, daidzin, quercitrin, rutin, and naringin.

15. The extract composition according to claim 13,
wherein the aglycone is apigenin.

16. A topical product or a food product, comprising:
the extract composition according to any one of claims 13 to 15, or a purified product thereof.
